# EUROPEAN PATENT APPLICATION

(11) **EP 1 113 008 A1**
(43) Date of publication of application: **04.07.2001**
(21) Application number: 00311164.8
(22) Date of filing: 14.12.2000
(51) Int. Cl.: C07D 239/22, C07D 403/06, C07D 403/12, A61P 31/04, A61P 33/02

(54) **4-oxo-2-ureido-1,4,5,6-tetrahydro-pyrimidine derivatives useful as antibacterial and antiprotozoal agents**

(30) Priority: 29.12.1999 US 173433 P
(71) Applicant: Pfizer Products Inc., Groton, Connecticut 06340 (US)
(72) Inventor: Linde II, Robert Gerald, Groton, Connecticut 06340 (US); Hayward, Matthew Merrill, Pfizer Global Res. & Dev, Groton, Connecticut 06340 (US); Kaneko, Takushi, Pfizer Global Res. and Dev., Groton, Connecticut 06340 (US)
(74) Representative: Wood, David John

(57) **Abstract**

The present invention relates to compounds of the formula **1** 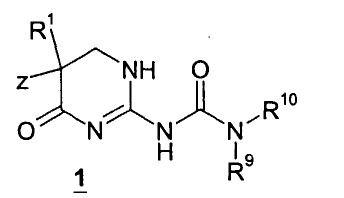 and to pharmaceutically acceptable salts, prodrugs and solvates thereof, wherein Z, R¹, R⁹, and R¹⁰ are as defined herein. The invention also relates to pharmaceutical compositions containing the above compounds and to methods of treating bacterial and protozoal infections in mammals by administering the above compounds.

## Description

### Background of the Invention

This invention relates to novel compounds that are useful as antibacterial and antiprotozoal agents in mammals, including man, as well as in fish and birds. This invention also relates to pharmaceutical compositions containing the novel compounds and to methods of treating bacterial and protozoal infections in mammals, fish and birds by administering the novel compounds to mammals, fish and birds requiring such treatment.

### Summary of the Invention

The present invention relates to compounds of the formula 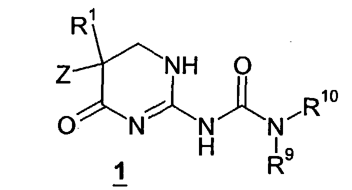 and to pharmaceutically acceptable salts, prodrugs and solvates thereof, wherein:
Z is a group having the following structure 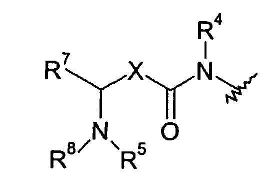 wherein X is -(CH₂)ₙ- and n is 0 or 1;
R¹ is selected from the differing α-carbon sidechain substituents on the naturally occurring amino acids selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine;
or R¹ is selected from the differing α-carbon sidechain substituents on the amino acids selected from hydroxylysine, demosine, isodemosine, 3-methylhistidine, norvalin, gamma-aminobutyric acid, citrulline, homocysteine, homoserine, ornithine and methionine sulfone;
or R¹ is selected from H, C₁-C₁₀ alkyl, C₂-C₁₀ alkenyl, C₂-C₁₀ alkynyl, -(CR⁴R⁵)ₜ(C₃-C₁₀ cycloalkyl), -(CR⁴R⁵)ₜ(C₆-C₁₀ aryl), -(CR⁴R⁵)ₜ(4 to 10 membered heterocyclic), -C(O)OR³, -C(O)NR³R⁴ and C(O)R³, wherein each t is independently an integer from 0 to 5, said alkyl, alkenyl and alkynyl groups optionally contain 1 or 2 hetero moieties selected from O, -S(O)ⱼwherein j is an integer from 0 to 2, and -N(R⁴)- with the proviso that two O atoms, two S atoms, or an O and S atom are not attached directly to each other, and the proviso that an O atom, a S atom or a N atom are not attached directly to a triple bond or non-aromatic double bond; said cycloalkyl, aryl and heterocyclic R¹ groups are optionally fused to a benzene ring, a C₅-C₈ cycloalkyl group, or a 4 to 10 membered heterocyclic group; the -(CR⁴R⁵)ₜ- moieties of the foregoing R¹ groups optionally include a carbon-carbon double or triple bond where t is an integer between 2 and 5; and the foregoing R¹ groups, except H but including any optional fused rings referred to above, are optionally substituted by 1 to 5 R² groups, and with the proviso that R¹ must be attached through a carbon atom unless R¹ is H; and with the proviso that if Z is 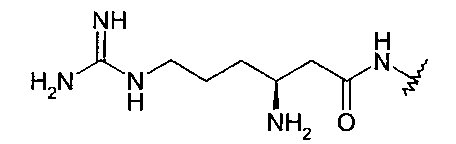 then R¹ cannot be H;
each R² is independently selected from C₁-C₁₀ alkyl, C₂-C₁₀ alkenyl, C₂-C₁₀ alkynyl, C₃-C₁₀ cycloalkyl, oxo, halo, cyano, nitro, trifluoromethyl, difluoromethoxy, trifluoromethoxy, azido, -OR³, -C(O)R³, -C(O)OR³, -NR⁴C(O)OR⁶, -OC(O)R³, -NR⁴SO₂R⁶, -SO₂NR³R⁴, -NR⁴C(O)R³, -C(O)NR³R⁴, -NR³R⁴, -S(O)ⱼ(CR⁴R⁵)ₘ(C₆-C₁₀ aryl), -S(O)ⱼ(C₁-C₆ alkyl), wherein j is an integer from 0 to 2, -(CR⁴R⁵)ₘ(C₆-C₁₀ aryl), -O(CR⁴R⁵)ₘ(C₆-C₁₀ aryl), -NR⁴(CR⁴R⁵)ₘ(C₆-C₁₀ aryl), and -(CR⁴R⁵)ₘ(4 to 10 membered heterocyclic), wherein each m is independently an integer from 0 to 4; said alkyl, alkenyl and alkynyl groups optionally contain 1 or 2 hetero moieties selected from O, -S(O)ⱼ- wherein j is an integer from 0 to 2, and -N(R³)- with the proviso that two O atoms, two S atoms, or an O and S atom are not attached directly to each other, and the proviso that an O atom, a S atom or a N atom are not attached directly to a triple bond or a non-aromatic double bond; said cycloalkyl, aryl and heterocyclic R² groups are optionally fused to a C₆-C₁₀ aryl group, a C₅-C₈ cycloalkyl group, or a 4 to 10 membered heterocyclic group; and said alkyl, cycloalkyl, aryl and heterocyclic R² groups are optionally substituted by 1 to 5 substituents independently selected from oxo, halo, cyano, nitro, trifluoromethyl, difluoromethoxy, trifluoromethoxy, azido, -NR⁴SO₂R⁶, -SO₂NR³R⁴, -C(O)R³, -C(O)OR³, -OC(O)R³, -NR⁴C(O)OR⁶, -NR⁴C(O)R³, -C(O)NR³R⁴, -NR³R⁴, -OR³, C₁-C₁₀ alkyl, -(CR⁴R⁵)ₘ(C₆-C₁₀ aryl), and -(CR⁴R⁵)ₘ(4 to 10 membered heterocyclic), wherein each m is independently an integer ranging from 0 to 4;
each R³ is independently selected from H, C₁-C₁₀ alkyl, -(CR⁴R⁵)ₘ(C₆-C₁₀ aryl), and -(CR⁴R⁵)ₘ(4 to 10 membered heterocyclic), wherein each m is independently an integer from 0 to 4; said alkyl group optionally includes 1 or 2 hetero moieties selected from O, -S(O)ⱼwherein j is an integer ranging from 0 to 2, and -N(R⁴)- with the proviso that two O atoms, two S atoms, or an O and S atom are not attached directly to each other; said cycloalkyl, aryl and heterocyclic R³ groups are optionally fused to a C₆-C₁₀ aryl group, a C₅-C₈ cycloalkyl group, or a 4 to 10 membered heterocyclic group; and the foregoing R³ substituents, except H, are optionally substituted by 1 to 5 substituents independently selected from oxo, halo, cyano, nitro, trifluoromethyl, difluoromethoxy, trifluoromethoxy, azido, -C(O)R⁴, -C(O)OR⁴, -OC(O)R⁴, -NR⁴C(O)R⁵, -C(O)NR⁴R⁵, -NR⁴R⁵, hydroxy, C₁-C₆ alkyl, and C₁-C₆ alkoxy, and with the proviso that R³ must be attached through a carbon atom unless R³ is H;
each R⁴ and R⁵ is independently H or C₁-C₆ alkyl;
each R⁶ is selected from the substituents provided in the definition of R³ except R⁶ is not H;
R⁷ is selected from the α-carbon substituents on the naturally occurring amino acids, as defined in R¹, as well as the α-carbon substituents on the amino acids selected from hydroxylysine, demosine, isodemosine, 3-methylhistidine, norvalin, citrulline, homocysteine, homoserine, ornithine and methionine sulfone;
R⁸ is H, C₁-C₆ alkyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), where t is an integer from 1 to 5;
R⁹ is independently H or C₁-C₆ alkyl; and,
R¹⁰ is H, C₁-C₆ alkyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), where t is an integer from 0 to 5.

As an example of R¹ or R⁷ as an α-carbon sidechain substituent on the naturally occurring amino acids, R¹ or R⁷ can be methyl or -(CH₂)₃NHC(NH)NH₂ from alanine or arginine, respectively.

In the first preferred embodiment of the compound of formula 1:
Z is 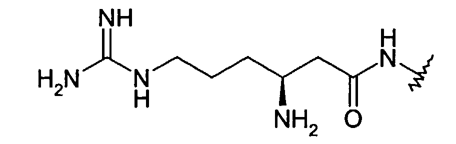 and R¹ is C₁-C₁₀ alkyl, C₂-C₁₀ alkenyl, C₂-C₁₀ alkynyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), wherein each t is independently an integer from 0 to 5, said alkyl, alkenyl and alkynyl groups optionally contain 1 or 2 hetero moieties selected from O, -S(O)ⱼ- wherein j is an integer from 0 to 2, and -N(R⁴)with the proviso that two O atoms, two S atoms, or an O and S atom are not attached directly to each other, and the proviso that an O atom, a S atom or a N atom are not attached directly to a triple bond or non-aromatic double bond; said cycloalkyl, aryl and heterocyclic R¹ groups are optionally fused to a benzene ring, a C₅-C₈ cycloalkyl group, or a 4 to 10 membered heterocyclic group; the -(CR⁴R⁵)ₜ- moieties of the foregoing R¹ groups optionally include a carbon-carbon double or triple bond where t is an integer between 2 and 5; and the foregoing R¹ groups, including any optional fused rings referred to above, are optionally substituted by 1 to 5 R² groups, and with the proviso that R¹ must be attached through a carbon atom; and R⁹ and R¹⁰ are as defined above.

In the second preferred embodiment of the compound of formula **1** the preferred groups are as indicated for the first preferred embodiment, but R⁹ and R¹⁰ are H.

Specific embodiments of the present invention include the following compounds as well as the pharmaceutically acceptable salts, prodrugs and solvates of the following compounds:
3S-Amino-6-guanidino-hexanoic acid methyl-(5S-methyl-4-oxo-2-ureido-1,4,5,6-tetrahydro-pyrimidin-5-yl)-amide;
3S-Amino-6-guanidino-hexanoic acid methyl-(5R-methyl-4-oxo-2-ureido-1,4,5,6-tetrahydro-pyrimidin-5-yl)-amide;
3S-Amino-6-guanidino-hexanoic acid (5S-ethyl-4-oxo-2-ureido-1,4,5,6-tetrahydro-pyrimidin-5-yl)-methyl-amide;
3S-Amino-6-guanidino-hexanoic acid (5R-ethyl-4-oxo-2-ureido-1,4,5,6-tetrahydro-pyrimidin-5-yl)-methyl-amide;
3S-Amino-6-guanidino-hexanoic acid (5S-hydroxymethyl-4-oxo-2-ureido-1,4,5,6-tetrahydro-pyrimidin-5-yl)-methyl-amide;
3S-Amino-6-guanidino-hexanoic acid (5R-hydroxymethyl-4-oxo-2-ureido-1,4,5,6-tetrahydro-pyrimidin-5-yl)-methyl-amide;
3S-Amino-6-guanidino-hexanoic acid (5R-fluoromethyl-4-oxo-2-ureido-1,4,5,6-tetrahydro-pyrimidin-5-yl)-methyl-amide;
3S-Amino-6-guanidino-hexanoic acid (5S-fluoromethyl-4-oxo-2-ureido-1,4,5,6-tetrahydro-pyrimidin-5-yl)-methyl-amide;
3S-Amino-6-guanidino-hexanoic acid [5S-(4-amino-butyl)-4-oxo-2-ureido-1,4,5,6-tetrahydro-pyrimidin-5-yl]-amide;
3S-Amino-6-guanidino-hexanoic acid [5R-(4-amino-butyl)-4-oxo-2-ureido-1,4,5,6-tetrahydro-pyrimidin-5-yl]-amide;
3S-Amino-6-guanidino-hexanoic acid [5S-(1 H-imidazol-4-ylmethyl)-4-oxo-2-ureido-1,4,5,6-tetrahydro-pyrimidin-5-yl]-amide;
3S-Amino-6-guanidino-hexanoic acid [5R-(1H-imidazol-4-ylmethyl)-4-oxo-2-ureido-1,4,5,6-tetrahydro-pyrimidin-5-yl]-amide;
3S-Amino-6-guanidino-hexanoic acid (5S-carbamoylmethyl-4-oxo-2-ureido-1,4,5,6-tetrahydro-pyrimidin-5-yl)-amide;
3S-Amino-6-guanidino-hexanoic acid (5R-carbamoylmethyl-4-oxo-2-ureido-1,4,5,6-tetrahydro-pyrimidin-5-yl)-amide;
3S,7-Diamino-heptanoic acid (5S-methyl-4-oxo-2-ureido-1,4,5,6-tetrahydro-pyrimidin-5-yl)-amide;
3S,7-Diamino-heptanoic acid (5R-methyl-4-oxo-2-ureido-1,4,5,6-tetrahydro-pyrimidin-5-yl)-amide;
3S-Amino-4-(1H-imidazol-4-yl)-N-(5R-methyl-4-oxo-2-ureido-1,4,5,6-tetrahydro-pyrimidin-5-yl)-butyramide;
3S-Amino-4-(1 H-imidazol-4-yl)-N-(5S-methyl-4-oxo-2-ureido-1,4,5,6-tetrahydro-pyrimidin-5-yl)-butyramide;
3R-Amino-4-(1 H-imidazol-4-yl)-N-(5R-methyl-4-oxo-2-ureido-1,4,5,6-tetrahydro-pyrimidin-5-yl)-butyramide;
3R-Amino-4-(1H-imidazol-4-yl)-N-(5S-methyl-4-oxo-2-ureido-1,4,5,6-tetrahydro-pyrimidin-5-yl)-butyramide;
3R-Amino-6-guanidino-hexanoic acid methyl-(5S-methyl-4-oxo-2-ureido-1,4,5,6-tetrahydro-pyrimidin-5-yl)-amide;
3R-Amino-6-guanidino-hexanoic acid methyl-(5R-methyl-4-oxo-2-ureido-1,4,5,6-tetrahydro-pyrimidin-5-yl)-amide;
3S-Amino-4-(1H-indol-3-yl)-N-(5R-methyl-4-oxo-2-ureido-1,4,5,6-tetrahydro-pyrimidin-5-yl)-butyramide;
3S-Amino-4-(1H-indol-3-yl)-N-(5S-methyl-4-oxo-2-ureido-1,4,5,6-tetrahydro-pyrimidin-5-yl)-butyramide;
2S-Amino-5-guanidino-pentanoic acid (5R-methyl-4-oxo-2-ureido-1,4,5,6-tetrahydro-pyrimidin-5-yl)-amide;
2S-Amino-5-guanidino-pentanoic acid (5S-methyl-4-oxo-2-ureido-1,4,5,6-tetrahydro-pyrimidin-5-yl)-amide;
2R-Amino-5-guanidino-pentanoic acid (5R-methyl-4-oxo-2-ureido-1,4,5,6-tetrahydro-pyrimidin-5-yl)-amide;
2R-Amino-5-guanidino-pentanoic acid (5S-methyl-4-oxo-2-ureido-1,4,5,6-tetrahydro-pyrimidin-5-yl)-amide;
3R-Amino-6-guanidino-hexanoic acid (5S-hydroxymethyl-4-oxo-2-ureido-1,4,5,6-tetrahydro-pyrimidin-5-yl)-methyl-amide;
3R-Amino-6-guanidino-hexanoic acid (5R-hydroxymethyl-4-oxo-2-ureido-1,4,5,6-tetrahydro-pyrimidin-5-yl)-methyl-amide;
3R-Amino-6-guanidino-hexanoic acid (5R-fluoromethyl-4-oxo-2-ureido-1,4,5,6-tetrahydro-pyrimidin-5-yl)-methyl-amide; and,
3R-Amino-6-guanidino-hexanoic acid (5S-fluoromethyl-4-oxo-2-ureido-1,4,5,6-tetrahydro-pyrimidin-5-yl)-methyl-amide.

The invention also relates to a pharmaceutical composition for the treatment of a disorder selected from a bacterial infection, a protozoal infection, and disorders related to bacterial infections or protozoal infections, in a mammal, fish, or bird which comprises a therapeutically effective amount of a compound of formula **1**, a prodrug thereof, a solvate thereof or a pharmaceutically acceptable salt thereof, and a pharmaceutically acceptable carrier.

The invention also relates to a pharmaceutical composition for the treatment of a disorder selected from a bacterial infection, a protozoal infection, and disorders related to bacterial infections or protozoal infections, in a mammal, fish, or bird which comprises a therapeutically effective amount of a compound of formula **1**, a prodrug thereof, a solvate thereof or a pharmaceutically acceptable salt thereof, in combination with a beta-lactam, quinolone, tetracycline, streptogramin, aminoglycoside, glycopeptide, macrolide or oxazolidinone antibiotic; or in combination with a compound which inhibits bacterial or protozoal efflux or degradation of a compound according to formula **1**.

The invention also relates to a method of treating a disorder selected from a bacterial infection, a protozoal infection, and disorders related to bacterial infections or protozoal infections, in a mammal, fish, or bird which comprises administering to said mammal, fish or bird a therapeutically effective amount of a compound of formula **1**, a prodrug thereof, a solvate thereof or a pharmaceutically acceptable salt thereof.

The invention also relates to a method of treating a disorder selected from a bacterial infection, a protozoal infection, and disorders related to bacterial infections or protozoal infections, in a mammal, fish, or bird which comprises administering to said mammal, fish or bird a therapeutically effective amount of a compound of formula **1**, a prodrug thereof, a solvate thereof or a pharmaceutically acceptable salt thereof, in combination or co-administered with a beta-lactam, quinolone, tetracycline, streptogramin, aminoglycoside, glycopeptide, macrolide or oxazolidinone antibiotic; or in combination with a compound which inhibits bacterial or protozoal efflux or degradation of a compound according to formula **1**.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, refers to the act of treating, as "treating" is defined immediately above.

As used herein, unless otherwise indicated, the terms or phrases "bacterial infection(s)", "protozoal infection(s)", and "disorders related to bacterial infections or protozoal infections" include but are not limited to the following: pneumonia, otitis media, sinusitus, bronchitis, tonsillitis, and mastoiditis related to infection by *Streptococcus pneumoniae, Haemophilus influenzae, Moraxella catarrhalis, Staphylococcus aureus, Enterococcus* faecalis, *E. faecium, E. casselflavus, S. epidermidis, S. haemolyticus,* or *Peptostreptococcus* spp.; pharyngitis, rheumatic fever, and glomerulonephritis related to infection by *Streptococcus pyogenes,* Groups C and G streptococci, *Corynebacterium diphtheriae,* or *Actinobacillus haemolyticum;* respiratory tract infections related to infection by *Mycoplasma pneumoniae, Legionella pneumophila, Streptococcus pneumoniae, Haemophilus influenzae,* or *Chlamydia pneumoniae;* blood and tissue infections, including endocarditis and osteomyelitis, caused by *S. aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracylines, oxazolidinones, and macrolides; uncomplicated skin and soft tissue infections and abscesses, and puerperal fever related to infection by *Staphylococcus aureus,* coagulase-negative staphylococci (i.e., *S. epidermidis, S. hemolyticus,* etc.), *Streptococcus pyogenes, Streptococcus agalactiae,* Streptococcal groups C-F (minute-colony streptococci), viridans streptococci, *Corynebacterium minutissimum, Clostridium* spp., or *Bartonella* henselae; uncomplicated acute urinary tract infections related to infection by *Staphylococcus aureus,* coagulase-negative staphylococcal species, or *Enterococcus* spp.; urethritis and cervicitis; sexually transmitted diseases related to infection by *Chlamydia trachomatis, Haemophilus ducreyi, Treponema pallidum, Ureaplasma urealyticum,* or *Neiserria gonorrheae;* toxin diseases related to infection by *S. aureus* (food poisoning and toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae, M. paratuberculosis, M. kansasii,* or *M. chelonei;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis or cardiovascular disease related to infection by *Helicobacter pylori* or *Chlamydia pneumoniae.* Bacterial infections and protozoal infections, and disorders related to such infections, which may be treated or prevented in animals include the following: bovine respiratory disease related to infection by *P. haemolytica, P. multocida, Mycoplasma bovis,* or *Bordetella* spp.; cow enteric disease related to infection by protozoa (i.e., coccidia, cryptosporidia, etc.); dairy cow mastitis related to infection by *S*. aureus, *Strep. uberis,* *Streptococcus agalactiae,* Streptococcus *dysgalactiae, Corynebacterium,* or- *Enterococcus* spp.; swine respiratory disease related to infection by *A. pleuro., P. multocida,* or *Mycoplasma* spp.; swine enteric disease related to infection by *Lawsonia intracellularis*, *Salmonella,* or *Serpulina hyodysinteriae;* cow footrot related to infection by *Fusobacterium* spp; cow hairy warts related to infection by *Fusobacterium necrophorum* or *Bacteroides nodosus;* cow pink-eye related to infection by *Moraxella* bovis; cow premature abortion related to infection by protozoa (i.e. neosporium); skin and soft tissue infections in dogs and cats related to infection by *S. epidermidis, S. intermedius,* coagulase neg. *Staphylococcus* or *P. multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas,* or *Prevotella.* Other bacterial infections and protozoal infections, and disorders related to such infections, which may be treated or prevented in accord with the method of the present invention are referred to in J. P. Sanford et al., "The Sanford Guide To Antimicrobial Therapy," 26th Edition, (Antimicrobial Therapy, Inc., 1996).

The compounds of the present invention may be active against the bacteria and protozoa, and associated conditions, referred to above, or specific strains of the bacteria and protozoa referred to above.

The term "halo", as used herein, unless otherwise indicated, includes fluoro, chloro, bromo or iodo. Preferred halo groups are fluoro and chloro.

The term "alkyl", as used herein, unless otherwise indicated, includes saturated monovalent hydrocarbon radicals having straight, cyclic or branched moieties. It is understood that for said alkyl group to include cyclic moieties it must contain at least three carbon atoms.

The term "alkenyl", as used herein, unless otherwise indicated, includes alkyl groups, as defined above, having at least one carbon-carbon double bond.

The term "alkynyl", as used herein, unless otherwise indicated, includes alkyl groups, as defined above, having at least one carbon-carbon triple bond.

The term "aryl", as used herein, unless otherwise indicated, includes an organic radical derived from an aromatic hydrocarbon by removal of one hydrogen, such as phenyl or naphthyl.

The term "differing α-carbon sidechain substituents on the naturally occuring amino acids", as used herein, unless otherwise indicated, are -CH₃ for alanine, -(CH₂)₃NHC(NH)NH₂ for arginine, -CH₂C(O)NH₂ for asparagine, -CH₂C(O)OH for aspartic acid, -CH₂SH for cysteine, -CH₂CH₂C(O)NH₂ for glutamine, -CH₂CH₂C(O)OH for glutamic acid, -H for glycine, -CH₂-imidazole for histidine, -CH(CH₃)CH₂CH₃ for isoleucine, -CH₂CH(CH₃)₂ for leucine, -(CH₂)₄NH₂ for lysine, -CH₂CH₂SCH₃ for methionine, -CH₂-phenyl for phenylalanine, -CH₂OH for serine, -CH(OH)CH₃ for threonine, -CH₂-indole for tryptophan, -CH₂-(4-phenol) for tyrosine and -CH(CH₃)₂ for valine. Other substituents are familiar to, or easily determined by, those skilled in the art.

The term "4 to 10 membered heterocyclic", as used herein, unless otherwise indicated, includes aromatic and non-aromatic heterocyclic groups containing one or more heteroatoms each selected from O, S and N, wherein each heterocyclic group has from 4 to 10 atoms in its ring system. Non-aromatic heterocyclic groups include groups having only 4 atoms in their ring system, but aromatic heterocyclic groups must have at least 5 atoms in their ring system. The heterocyclic groups include benzo-fused ring systems and ring systems substituted with one or more oxo moieties. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6tetrahydropyridinyl, 2-pyrrolinyl, 3-pyrrolinyl, indolinyl, 2H-pyranyl, 4H-pyranyl, dioxanyl, 1,3dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl (N-attached) or pyrrol-3-yl (C-attached). The terms "5 to 12 membered heterocyclic", "5 to 6 membered heterocyclic", and other uses of "heterocyclic", correspond to the above definition with an appropriate number of ring members.

The term "Me" means methyl, "Et" means ethyl, and "Ac" means acetyl.

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds of are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include a basic moiety, such as an amino group, may form pharmaceutically acceptable salts with various amino acids, in addition to the acids mentioned above.

Those compounds of the present invention that are acidic in nature are capable of forming base salts with various pharmacologically acceptable cations. Examples of such salts include the alkali metal or alkaline earth metal salts and, particularly, the calcium, magnesium, sodium and potassium salts of the compounds of the present invention.

The compounds of the present invention have asymmetric centers and therefore exist in different enantiomeric and diastereomeric forms. This invention relates to the use of all optical isomers and stereoisomers of the compounds of the present invention, and mixtures thereof, and to all pharmaceutical compositions and methods of treatment that may employ or contain them. The compounds of formula **1** may also exist as tautomers. This invention relates to the use of all such tautomers and mixtures thereof.

The subject invention also includes isotopically-labelled compounds, and the pharmaceutically acceptable salts thereof, which are identical to those recited in formula **1**, but for the fact that one or more atoms are replaced by an atom having an atomic mass or mass number different from the atomic mass or mass number usually found in nature. Examples of isotopes that can be incorporated into, compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula **1** of this invention and prodrugs thereof can generally be prepared by carrying out the procedures disclosed in the Schemes and/or in the Examples and Preparations below, by substituting a readily available isotopically labelled reagent for a non-isotopically labelled reagent.

This invention also encompasses pharmaceutical compositions containing and methods of treating bacterial infections through administering prodrugs of compounds of the formula **1**. Compounds of formula **1** having free amino, amido, hydroxy or carboxylic groups can be converted into prodrugs. Prodrugs include compounds wherein an amino acid residue, or a polypeptide chain of two or more (e.g., two, three or four) amino acid residues is covalently joined through an amide or ester bond to a free amino, hydroxy or carboxylic acid group of compounds of formula **1**. The amino acid residues include but are not limited to the 20 naturally occurring amino acids commonly designated by three letter symbols and also includes 4-hydroxyproline, hydroxylysine, demosine, isodemosine, 3-methylhistidine, norvalin, beta-alanine, gamma-aminobutyric acid, citrulline homocysteine, homoserine, omithine and methionine sulfone. Additional types of prodrugs are also encompassed. For instance, free carboxyl groups can be derivatized as amides or alkyl esters. Free hydroxy groups may be derivatized using groups including but not limited to hemisuccinates, phosphate esters, dimethylaminoacetates, and phosphoryloxymethyloxycarbonyls, as outlined in *Advanced Drug Delivery Reviews,* **1996**, *19*, 115. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers wherein the acyl group may be an alkyl ester, optionally substituted with groups including but not limited to ether, amine and carboxylic acid functionalities, or where the acyl group is an amino acid ester as described above, are also encompassed. Prodrugs of this type are described in *J. Med. Chem.* **1996**, *39*, 10. Free amines can also be derivatized as amides, sulfonamides or phosphonamides. All of these prodrug moieties may incorporate groups including but not limited to ether, amine and carboxylic acid functionalities.

### Detailed Description of the Invention

The preparation of the compounds of the present invention is described and illustrated below.

Preparation of the compounds of formula **1** can most flexibly be carried out through assembly of Fragments A and B as outlined below.

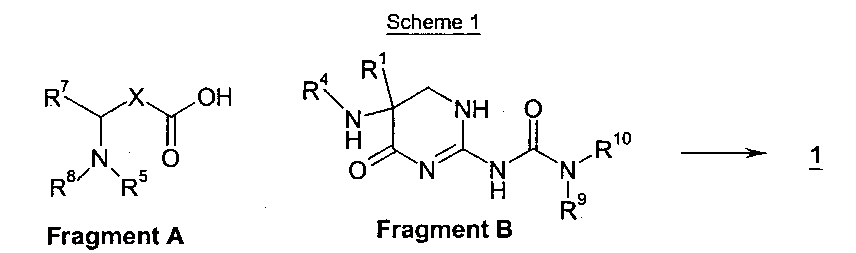

### Coupling of Fragment A and Fragment B

Following the precedent of *J. Am. Chem Soc.,* **1997,** *119*, 11777 and *Eur. J. Org. Chem.,* **1998**, 777 the two fragments may be coupled by using an amide coupling agent such as bis(-2-oxo-3-oxazolidinyl)phosphinic chloride (BOP-CI), 2-ethoxy-1-ethoxycarbonyl-1,2-dihydroquinoline (EEDQ), 1,1'-carbonyl-diimidazole (CDI), or a carbodiimide such as dicyclohexylcarbodiimide (DCC).

### Preparation of Fragment A

The compounds represented by Fragment A, when n = 0 and R⁵ and R⁸ are H and R⁷ is as defined above, are commercially available. It may be advantageous'to protect the amine(s) or other nitrogen functionalities as their 9-fluorenylmethoxycarbonyl- (FMOC), benzyloxycarbonyl- (CBZ) or tert-butoxycarbonyl (BOC) carbamates (see *Protective Groups in Organic Synthesis,* T. Greene and P. Wuts, Eds., John Wiley & Sons Ltd., New York, 1991 or *Protecting Groups,* P. Kocienski, Ed., Thieme Medical Publishers, New York, 1994) and subsequently deprotect at an appropriate time.

Fragment A, when n = 0 and R⁵ is H, R⁸ is lower alkyl and R⁷ is as defined, may be prepared by reductive alkylation of the appropriate commercially available amino acid. Combination of the amine and the appropriate aldehyde in a solvent such as methanol or ethanol and treatment with a reducing agent such as sodium borohydride (NaBH₄), sodium triacetoxyborohydride (NaBH(OAc)₃) or sodium cyanoborohydride (NaCNBH₃) at a temperature ranging from 0°C to 50°C provides the product. The resultant secondary amine may be protected, as described above, or reacted in a second reductive alkylation reaction to give the compound where n = 0 and R⁵ and R⁸ are independently lower alkyl and R⁷ is as defined.

Fragment A, when n = 1, R⁵ and R⁸ are H and R⁷ is as defined, may be prepared by homologating the corresponding commercially available amino acid (where n = 0) according to the precedent in *J. Am. Chem.* Soc., **1997,** *119,* 11777 and *Eur. J. Org. Chem.,* **1998,** 777. It may be advantageous to protect the amine as above prior to coupling with Fragment B.

Fragment A, when n = 1, at least one of R⁵ or R⁸ is not H and R⁷ is as defined, may be prepared by homologation followed by reductive alkylation, as above.

### Preparation of Fragment B

Preparation of Fragment B, where R¹ is H and R⁴ is as defined, can be prepared using the chemistries described in *Eur. J. Org. Chem.,* **1998,** 777. If R⁴ is other than H or methyl then R⁴ may be introduced as its aldehyde, replacing formaldehyde in the chemistry presented in the above reference.

Preparation of Fragment B, where R¹ is as defined but not H and R⁴ is as defined, can most flexibly be carried out through assembly of Fragments C and guanylurea as outlined below based on the precedent of *J. Org. Chem.,* **1987**, 52, 4007. L is a leaving group such as -O-tosyl (-OTs), -O-mesyl (-OMs) or halide.

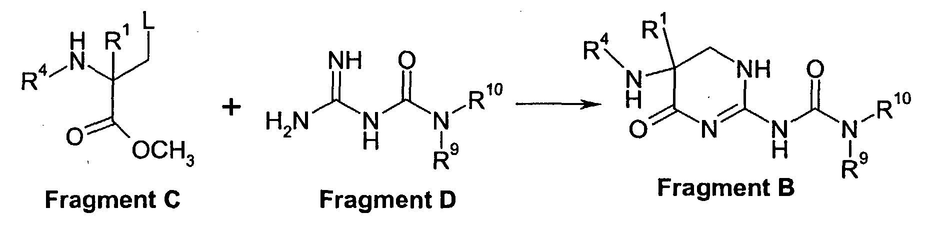

Fragment B may also be assembled based on the chemistries in *J. Am. Chem. Soc.,* **1997**, *119,* 11777 wherein Fragment C, as shown below with L = NH₂, is coupled with Fragment E, prepared as in said reference. Fragment C may be prepared utilizing the chemistry in the above reference and modifying as appropriate using methods known to one skilled in the art.

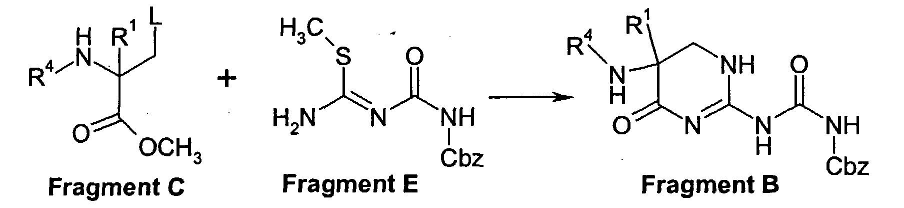

### Preparation of Fragment C

Fragment C, where L = OTs, R⁴ is as defined and R¹ is selected from the α-carbon substituents on the naturally occurring amino acids, may be prepared by first forming compound **2** according to the precedent in *Arch. Biochem. Biophys.,* **1960**, *90*, 254 from the appropriate amino acid (either D or L).

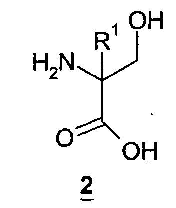

Compound **2** may then be elaborated into Fragment C based on the precedent in *Eur. J. Org. Chem.,* **1998**, 777.

Fragment C, where L = OTs, R⁴ is as defined and R¹ is as defined but not selected from the α-carbon substituents on the naturally occurring amino acids, may be prepared from D- or L-serine. Formation of compound 3 (see *Eur. J. Org. Chem.,* **1998**, 777) followed by displacement of the tosylate with the appropriate nucleophile, for example, a Grignard, organolithium or organocerium reagent *(Tetrahedron Lett.,* **1984** *25*, 4233), in a solvent such as tetrahydrofuran (THF), dioxane or diethyl ether (Et₂O) at a temperature ranging from -78°C to 25°C may afford compound **4**. These reagents may be prepared from the corresponding halide using standard procedures (see *Organometallics In Synthesis; A Manual,* M. Schlosser, Ed., John Wiley & Sons Ltd., New York, 1994). Compound **4** may then be elaborated to Fragment C using the chemistries precedented in *Arch. Biochem. Biophys.,* **1960**, *90*, 254 followed by tosylation of the resultant primary alcohol, for example, by reaction with tosyl chloride in an aprotic solvent, such as methylene chloride or dimethylformamide, and in the presence of an amine base such as pyridine or triethylamine.

Fragment C, where R¹ is -CH₂-NR₃R₄ may by prepared by reductive amination, as above, with the appropriate amine (HNR₃R₄) to the aldehyde **5**, generated from oxidation of **6** by Swern (*J*. *Org. Chem.,* **1976,** *41,* 3329) or Dess-Martin (*J. Org. Chem*., **1983,** *48,* 4155) conditions. Compound 6 is an intermediate in the preparation of compound **3**. The resulting amine may then be elaborated to Fragment C using the chemistries precedented in *Arch. Biochem. Biophys.,* **1960**, *90*, 254 followed by tosylation of the resultant primary alcohol, for example, by reaction with tosyl chloride in an aprotic solvent, such as methylene chloride or dimethylformamide, and in the presence of an amine base such as pyridine or triethylamine.

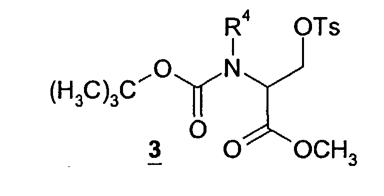

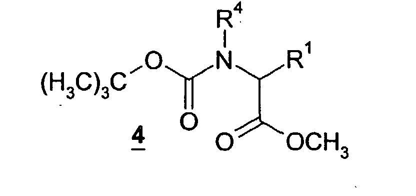

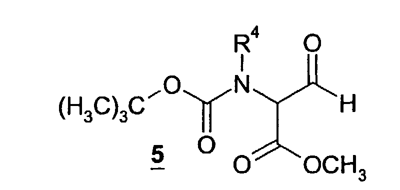

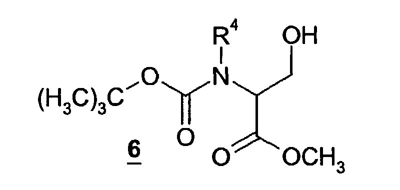

Fragment C, where R¹ = -COR³, may be prepared by addition of the appropriate R³ nucleophile to **5** followed by oxidation (see above references). The resulting ketone may then be elaborated to Fragment C using the chemistries precedented in *Arch. Biochem. Biophys.,* **1960**, *90*, 254 followed by tosylation of the resultant primary alcohol, for example, by reaction with tosyl chloride in an aprotic solvent, such as methylene chloride or dimethylformamide, and in the presence of an amine base such as pyridine or triethylamine.

Fragment C, where R¹ = -COOR³ or -CONR³R⁴, may be prepared by oxidation of **5** to the acid, for example through the action of potassium permanganate *(Tetrahedron Lett.,* 1986, *27,* 4537 and *J. Am. Chem. Soc.,* **1987,** *109*, 7575) or sodium chlorite (*J. Org. Chem.* **1989,** *54,* 4100), followed by esterification with HOR³ or amidation with HNR³R⁴, for example in the presence of a coupling agent such as 1-(3-dimethylaminopropyl)-3-ethylcarbodiimide hydrochloride (EDC), diethyl pyrocarbonate (DEPC), DCC, CDI or EEDQ in a solvent such as dichloromethane, DMF or chloroform at a temperature ranging from 0°C to 25°C. The resulting ester or amide may then be elaborated to Fragment C as above.

Fragment C, where R¹ = aryl, heteroaryl, allyl or vinyl, may be prepared by reaction of the α-bromide of di-BOC-glycine with the appropriate Grignard reagent, R¹-MgBr, based on the precedent in *Syn.,* **1987**, *3*, 223. This coupled product may be deprotected with trifluoroacetic acid and then elaborated as above to Fragment C.

In the above chemistries it may be advantageous to protect nitrogen functionalities as their tert-butoxycarbonyl carbamates (see above) prior to coupling with guanylurea and deprotecting, for example with trifluoroacetic acid, prior to coupling with Fragment A.

If in any of the above chemistries the methyl ester in Fragment C is not compatible then the carboxylic acid may be masked as a different, more stable, ester. For example, a tert-butyl ester may be introduced instead of the methyl ester using standard chemistry (see *Protective Groups in Organic Synthesis, T.* Greene and P. Wuts, Ed., John Wiley & Sons Ltd., New York, 1991 or *Protecting Groups,* P. Kocienski, Ed., Thieme Medical Publishers, New York, 1994) and deprotected and the methyl ester reintroduced prior to coupling with guanyl urea. The ester may also be kept as its carboxylic acid until prior to coupling where the methyl ester could be introduced.

### Preparation of Fragment D

Fragment D, where R⁹ and R¹⁰ are H is commercially available. Fragment D, where R⁹ is H and R¹⁰ is as defined may be prepared by using the methods described in *Can. J. Chem.,* **1954,** *32,* 242, *J. Amer. Chem. Soc.,* **1959,** *81,* 2220 and *Bull. Acad. Pol. Sci. Ser. Sci. Chim.,* **1953,** 74. Fragment D, where R⁹ is as defined and R¹⁰ is as defined may be prepared by using the methods described in *Arzneim. Forsch*., **1978,** *28,* 1435 or by modification, by one skilled in the art, of the above references for Fragment D, when R⁹ is H and R¹⁰ is as defined.

In the foregoing description, compounds may contain R¹ groups that may not be compatible with the described chemistries. Functional groups within R¹ that are not compatible with chemistry utilized may be protected. For example, an alcohol might be protected as an ether (benzyl, allyl or silyl) or ester (benzoate, pivaloate or acetate) and subsequently deprotected at an appropriate time. If R¹ contains a ketone it may be necessary to protect it, for instance as a dimethyl ketal through the use of methanol and catalytic acid such as camphorsulfonic acid or p-toluenesulfonic acid (p-TsOH). Deprotection of the ketal using aqueous acid can be carried out at a later time. Alternatively, a ketone may be masked as its protected alcohol, which can then be regenerated by deprotection and oxidation, for example under Swern conditions (*J*. *Org. Chem.,* **1976**, *41*, 3329). An amine might be protected as its 9-fluorenylmethoxycarbonyl- (FMOC), benzyloxycarbonyl- (CBZ) or tert-butoxycarbonyl (BOC) carbamates (see *Protective Groups in Organic Synthesis, T.* Greene and P. Wuts, Ed., John Wiley & Sons Ltd., New York, 1991 or *Protecting Groups, P.* Kocienski, Ed., Thieme Medical Publishers, New York, 1994) and subsequently deprotected at an appropriate time. It might also be advantageous to introduce said groups at a later stage by utilizing an intermediate that may, at an appropriate time, be further elaborated to the desired R¹. Acids, carbonyl-linked amides and esters may be generated from a protected primary alcohol, which is unmasked by deprotection and elaborated by double oxidation, for example Swern conditions followed by action of potassium permanganate (*Tetrahedron Lett.,* **1986,** *27,* 4537 and *J. Am. Chem. Soc.,* **1987,** 109, 7575), or sodium chlorite (*J. Org. Chem.,* **1986,** *51,* 567 and *J. Am. Chem.* Soc., **1997,** *119,* 7974) to the carboxylic acid. This may then coupled with the appropriate alcohol or amine, for instance by the action of DCC, to produce the desired ester or amide. An N-linked amide or sulfonamide may be carried through as an amine, protected as above, which is then deprotected and acylated or sulfonylated. N-linked amides and sulfonamides and amines may alternatively be introduced by displacement of a leaving group. For example, a protected alcohol may be deprotected and the resulting alcohol transformed into the mesylate, for instance through the action of methanesulfonyl chloride and triethylamine (NEt₃) (*J*. *Org. Chem.,* **1970,** *35*, 3195). The mesylate is then displaced by azide, for example using sodium azide in N,N-dimethylformamide (DMF) and the azide reduced to the primary amine using for instance triphenylphosphine followed by aqueous hydrolysis. Acylation may then provide the corresponding amide. Sulfur-containing moieties may also be introduced in this fashion, for example, by the displacement of the aforementioned mesylate with the appropriate thiolate or protected thiolate, followed if necessary by oxidation of the sulfur to the sulfoxide or sulfone.

The compounds of the present invention have asymmetric carbon atoms. Compounds having a mixture of isomers at one or more centers will exist as diastereomeric mixtures, which can be separated into their individual diastereomers on the basis of their physical chemical differences by methods known to those skilled in the art, for example, by chromatography or fractional crystallization. All such isomers, including diastereomer mixtures, are considered as part of the invention.

The compounds of the present invention that are basic in nature are capable of forming a wide variety of different salts with various inorganic and organic acids. Although such salts must be pharmaceutically acceptable for administration to animals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the basic compounds of this invention are readily prepared by treating the basic compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of the present invention that are acidic in nature, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium, calcium and magnesium, etc. These salts can easily be prepared by treating the corresponding acidic compounds with an aqueous solution containing the desired alkali metal alkoxide or metal hydroxide, and then evaporating the resulting solution to dryness, preferably under reduced pressure. Alternatively, they may also be prepared by mixing lower alkanolic solutions of the acidic compounds and the desired alkali metal alkoxide or metal hydroxide together, and then evaporating the resulting solution to dryness in the same manner as before. In either case, stoichiometric quantities of reagents are preferably employed in order to ensure completeness of reaction and maximum yields of the desired final product.

The antibacterial activity of the compounds of the present invention against bacterial pathogens is demonstrated by the compound's ability to inhibit growth of defined strains of pathogens.

### Assay

The assay, described below, employs conventional methodology and interpretation criteria and is designed to provide direction for chemical modifications that may lead to compounds with antibacterial activity against susceptible and drug-resistant organisms including, but not limited to, beta-lactam, macrolide and vancomycin resistance. In the assay, a panel of bacterial strains is assembled to include a variety of target pathogenic species, including representatives of antibiotic resistant bacteria. Use of this panel enables the chemical structure/activity relationship to be determined with respect to potency and spectrum of activity. The assay is performed in microtiter trays and interpreted according to Performance Standards for Antimicrobial. Disk Susceptibility Tests - Sixth Edition; Approved Standard, published by The National Committee for Clinical Laboratory Standards (NCCLS) guidelines; the minimum inhibitory concentration (MIC) is used to compare strains. Compounds are initially dissolved in dimethylsulfoxide (DMSO) as stock solutions.

The activity of the compounds of the present invention also may be assessed in accord with Steers replicator technique which is a standard in vitro bacterial testing method described by Steers et al., *Antibiotics and Chemotherapy* **1959,** 9, 307.

The in vivo activity of the compounds of the present invention can be determined by conventional animal protection studies well known to those skilled in the art, usually carried out in rodents.

According to one *in vivo* model, compounds are evaluated for efficacy in mouse models of acute bacterial infection. An example of one such *in vivo* system is provided as follows. Mice (CF1 mixed sex mice; 18-20 g) are allotted to cages upon their arrival, and allowed to acclimate 1-2 days before being placed in a study. The acute infection is produced by intraperitoneal inoculation of bacteria (*Staphylococcus aureus* strain 01A1095) suspended in 5% sterile hog gastric mucin. The inoculum is prepared by: growing the culture overnight at 37°C on blood agar, harvesting the resulting surface growth with sterile brain heart infusion broth, and adjusting this suspension to a turbidity that when diluted 1:10 into 5% sterile hog gastric mucin would produce 100% lethality.

Mice (10 per group) are treated subcutaneously, at 0.5 hour and 4 hours after challenge. Appropriate non-treated (infected but not treated) and positive (vancomycin or minocycline, etc.) controls are included in each study. Percent survival is recorded after a 4day observation period; the PD₅₀ (mg/kg/dose calculated to protect 50% of infected animals) is determined by the probit method.

The compounds of the present invention, and the pharmaceutically acceptable salts thereof (hereinafter "the active compounds"), may be administered through oral, parenteral, topical, inhaled, or rectal routes in the treatment of bacterial and protozoal infections. In general, these compounds are most desirably administered in dosages ranging from about 0.2 mg per kg body weight per day (mg/kg/day) to about 200 mg/kg/day in single or divided doses (i.e., from 1 to 4 doses per day), although variations will necessarily occur depending upon the species, weight and condition of the subject being treated and the particular route of administration chosen. However, a dosage level that is in the range of about 3 mg/kg/day to about 60 mg/kg/day is most desirably employed. Variations may nevertheless occur depending upon the species of mammal, fish or bird being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds may be administered alone or in combination with pharmaceutically acceptable carriers or diluents by the routes previously indicated, and such administration may be carried out in single or multiple doses. More particularly, the active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral adinistration, the active compound may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound in either sesame or peanut oil or in aqueous ethanol or propylene glycol may be employed. Use of a cyclodextrin derivative such as β-cyclodextrin sulfobutyl ether, sodium salt (see United, States patent 5,134,127) may also be advantageous. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques known to those skilled in the art.

Additionally, it is also possible to administer the active compounds of the present invention topically and this may be done by way of creams, jellies, gels, pastes, patches, ointments and the like, in accordance with standard pharmaceutical practice.

For administration to animals other than humans, such as cattle or domestic animals, the active compounds may be administered in the feed of the animals or orally as a drench composition.

The active compounds may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoyl residues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

## Claims

1. A compound of the formula 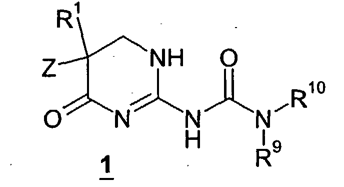 or a pharmaceutically acceptable salt, prodrug or solvate thereof, wherein:
Z is a group having the following structure 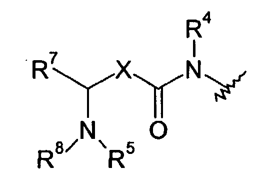 wherein X is-(CH₂)ₙ- and n is 0 or 1;
R¹ is selected from the differing α-carbon sidechain substituents on the naturally occurring amino acids selected from alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine;
or R¹ is selected from the differing α-carbon sidechain substituents on the amino acids selected from hydroxylysine, demosine, isodemosine, 3-methylhistidine, norvalin, gamma-aminobutyric acid, citrulline, homocysteine, homoserine, ornithine and methionine sulfone;
or R¹ is selected from H, C₁-C₁₀ alkyl, C₂-C₁₀ alkenyl, C₂-C₁₀ alkynyl, -(CR⁴R⁵)ₜ(C₃-C₁₀ cycloalkyl), -(CR⁴R⁵)ₜ(C₆-C₁₀ aryl), -(CR⁴R⁵)ₜ(4 to 10 membered heterocyclic), -C(O)OR³, -C(O)NR³R⁴ and C(O)R³, wherein each t is independently an integer from 0 to 5, said alkyl, alkenyl and alkynyl groups optionally contain 1 or 2 hetero moieties selected from O, -S(O)ⱼ- wherein j is an integer from 0 to 2, and -N(R⁴)- with the proviso that two O atoms, two S atoms, or an O and S atom are not attached directly to each other, and the proviso that an O atom, a S atom or a N atom are not attached directly to a triple bond or non-aromatic double bond; said cycloalkyl, aryl and heterocyclic R¹ groups are optionally fused to a benzene ring, a C₅-C₈ cycloalkyl group, or a 4 to 10 membered heterocyclic group; the -(CR⁴R⁵)ₜ- moieties of the foregoing R¹ groups optionally include a carbon-carbon double or triple bond where t is an integer between 2 and 5; and the foregoing R¹ groups, except H but including any optional fused rings referred to above, are optionally substituted by 1 to 5 R² groups, and with the proviso that R¹ must be attached through a carbon atom unless R¹ is H; and with the proviso that if Z is 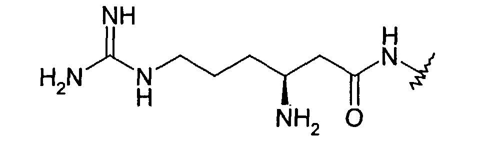 then R¹ cannot be H;
each R² is independently selected from C₁-C₁₀ alkyl, C₂-C₁₀ alkenyl, C₂-C₁₀ alkynyl, C₃-C₁₀ cycloalkyl, oxo, halo, cyano, nitro, trifluoromethyl, difluoromethoxy, trifluoromethoxy, azido, -OR³, -C(O)R³, -C(O)OR³, -NR⁴C(O)OR⁶, -OC(O)R³, -NR⁴SO₂R⁶, -SO₂NR³R⁴, -NR⁴C(O)R³, -C(O)NR³R⁴, -NR³R⁴, -S(O)ⱼ(CR⁴R⁵)ₘ(C₆-C₁₀ aryl), -S(O)ⱼ(C₁-C₆ alkyl), wherein j is an integer from 0 to 2, -(CR⁴R⁵)ₘ(C₆-C₁₀ aryl), -O(CR⁴R⁵)ₘ(C₆-C₁₀ aryl), -NR⁴(CR⁴R⁵)ₘ(C₆-C₁₀ aryl), and -(CR⁴R⁵)ₘ(4 to 10 membered heterocyclic), wherein each m is independently an integer from 0 to 4; said alkyl, alkenyl and alkynyl groups optionally contain 1 or 2 hetero moieties selected from O, -S(O)ⱼ- wherein j is an integer from 0 to 2, and -N(R³)- with the proviso that two O atoms, two S atoms, or an O and S atom are not attached directly to each other, and the proviso that an O atom, a S atom or a N atom are not attached directly to a triple bond or a non-aromatic double bond; said cycloalkyl, aryl and heterocyclic R² groups are optionally fused to a C₆-C₁₀ aryl group, a C₅-C₈ cycloalkyl group, or a 4 to 10 membered heterocyclic group; and said alkyl, cycloalkyl, aryl and heterocyclic R² groups are optionally substituted by 1 to 5 substituents independently selected from oxo, halo, cyano, nitro, trifluoromethyl, difluoromethoxy, trifluoromethoxy, azido, -NR⁴SO₂R⁶, -SO₂NR³R⁴, -C(O)R³, -C(O)OR³, -OC(O)R³, -NR⁴C(O)OR⁶, -NR⁴C(O)R³, -C(O)NR³R⁴, -NR³R⁴, -OR³, C₁-C₁₀ alkyl, -(CR⁴R⁵)ₘ(C₆-C₁₀ aryl), and -(CR⁴R⁵)ₘ(4 to 10 membered heterocyclic), wherein each m is independently an integer ranging from 0 to 4;
each R³ is independently selected from H, C₁-C₁₀ alkyl, -(CR⁴R⁵)ₘ(C₆-C₁₀ aryl), and -(CR⁴R⁵)ₘ(4 to 10 membered heterocyclic), wherein each m is independently an integer from 0 to 4; said alkyl group optionally includes 1 or 2 hetero moieties selected from O, -S(O)ⱼ - wherein j is an integer ranging from 0 to 2, and -N(R⁴)- with the proviso that two O atoms, two S atoms, or an O and S atom are not attached directly to each other; said cycloalkyl, aryl and heterocyclic R³ groups are optionally fused to a C₆-C₁₀ aryl group, a C₅-C₈ cycloalkyl group, or a 4 to 10 membered heterocyclic group; and the foregoing R³ substituents, except H, are optionally substituted by 1 to 5 substituents independently selected from oxo, halo, cyano, nitro, trifluoromethyl, difluoromethoxy, trifluoromethoxy, azido, -C(O)R⁴, -C(O)OR⁴, -OC(O)R⁴, -NR⁴C(O)R⁵, -C(O)NR⁴R⁵, -NR⁴R⁵, hydroxy, C₁-C₆ alkyl, and C₁-C₆ alkoxy, and with the proviso that R³ must be attached through a carbon atom unless R³ is H;
each R⁴ and R⁵ is independently H or C₁-C₆ alkyl;
each R⁶ is selected from the substituents provided in the definition of R³ except R⁶ is not H;
R⁷ is selected from the α-carbon substituents on the naturally occurring amino acids, as defined in R¹, as well as the α-carbon substituents on the amino acids selected from hydroxylysine, demosine, isodemosine, 3-methylhistidine, norvalin, citrulline, homocysteine, homoserine, ornithine and methionine sulfone;
R⁸ is H, C₁-C₆ alkyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), where t is an integer from 1 to 5;
R⁹ is independently H or C₁-C₆ alkyl; and,
R¹⁰ is H, C₁-C₆ alkyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), where t is an integer from 0 to 5.

2. A compound according to claim 1 wherein Z is 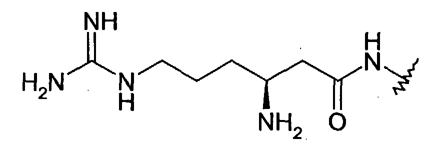 and R¹ is C₁-C₁₀ alkyl, C₂-C₁₀ alkenyl, C₂-C₁₀ alkynyl, -(CR⁴R⁵)ₜ(C₃-C₁₀ cycloalkyl), -(CR⁴R⁵)ₜ(C₆-C₁₀ aryl), or -(CR⁴R⁵)ₜ(4 to 10 membered heterocyclic), wherein each t is independently an integer from 0 to 5, said alkyl, alkenyl and alkynyl groups optionally contain 1 or 2 hetero moieties selected from O, -S(O)ⱼ- wherein j is an integer from 0 to 2, and -N(R⁴) - with the proviso that two O atoms, two S atoms, or an O and S atom are not attached directly to each other, and the proviso that an O atom, a S atom or a N atom are not attached directly to a triple bond or non-aromatic double bond; said cycloalkyl, aryl and heterocyclic R¹ groups are optionally fused to a benzene ring, a C₅-C₈ cycloalkyl group, or a 4 to 10 membered heterocyclic group; the -(CR⁴R⁵)ₜ- moieties of the foregoing R¹ groups optionally include a carbon-carbon double or triple bond where t is an integer between 2 and 5; and the foregoing R¹ groups, including any optional fused rings referred to above, are optionally substituted by 1 to 5 R² groups, and with the proviso that R¹ must be attached through a carbon atom.

3. A compound according to claim 2 wherein R⁹ and R¹⁰ are both H.

4. A compound according to claim 1 wherein said compound is selected from the following compounds as well as the pharmaceutically acceptable salts, prodrugs and solvates of the following compounds:
3S-Amino-6-guanidino-hexanoic acid methyl-(5S-methyl-4-oxo-2-ureido-1,4,5,6-tetrahydro-pyrimidin-5-yl)-amide;
3S-Amino-6-guanidino-hexanoic acid methyl-(5R-methyl-4-oxo-2-ureido-1,4,5,6-tetrahydro-pyrimidin-5-yl)-amide;
3S-Amino-6-guanidino-hexanoic acid (5S-ethyl-4-oxo-2-ureido-1,4,5,6-tetrahydro-pyrimidin-5-yl)-methyl-amide;
3S-Amino-6-guanidino-hexanoic acid (5R-ethyl-4-oxo-2-ureido-1,4,5,6-tetrahydro-pyrimidin-5-yl)-methyl-amide;
3S-Amino-6-guanidino-hexanoic acid (5S-hydroxymethyl-4-oxo-2-ureido-1,4,5,6-tetrahydro-pyrimidin-5-yl)-methyl-amide;
3S-Amino-6-guanidino-hexanoic acid (5R-hydroxymethyl-4-oxo-2-ureido-1,4,5,6-tetrahydro-pyrimidin-5-yl)-methyl-amide;
3S-Amino-6-guanidino-hexanoic acid (5R-fluoromethyl-4-oxo-2-ureido-1,4,5,6-tetrahydro-pyrimidin-5-yl)-methyl-amide;
3S-Amino-6-guanidino-hexanoic acid (5S-fluoromethyl-4-oxo-2-ureido-1,4,5,6-tetrahydro-pyrimidin-5-yl)-methyl-amide;
3S-Amino-6-guanidino-hexanoic acid [5S-(4-amino-butyl)-4-oxo-2-ureido-1,4,5,6-tetrahydro-pyrimidin-5-yl]-amide;
3S-Amino-6-guanidino-hexanoic acid [5R-(4-amino-butyl)-4-oxo-2-ureido-1,4,5,6-tetrahydro-pyrimidin-5-yl]-amide;
3S-Amino-6-guanidino-hexanoic acid [5S-(1H-imidazol-4-ylmethyl)-4-oxo-2-ureido-1,4,5,6-tetrahydro-pyrimidin-5-yl]-amide;
3S-Amino-6-guanidino-hexanoic acid [5R-(1 H-imidazol-4-ylmethyl)-4-oxo-2-ureido-1,4,5,6-tetrahydro-pyrimidin-5-yl]-amide;
3S-Amino-6-guanidino-hexanoic acid (5S-carbamoylmethyl-4-oxo-2-ureido-1,4,5,6-tetrahydro-pyrimidin-5-yl)-amide;
3S-Amino-6-guanidino-hexanoic acid (5R-carbamoylmethyl-4-oxo-2-ureido-1,4,5,6-tetrahydro-pyrimidin-5-yl)-amide;
3S,7-Diamino-heptanoic acid (5S-methyl-4-oxo-2-ureido-1,4,5,6-tetrahydro-pyrimidin-5-yl)-amide;
3S,7-Diamino-heptanoic acid (5R-methyl-4-oxo-2-ureido-1,4,5,6-tetrahydro-pyrimidin-5-yl)-amide;
3S-Amino-4-(1 H-imidazol-4-yl)-N-(5R-methyl-4-oxo-2-ureido-1,4,5,6-tetrahydro-pyrimidin-5-yl)-butyramide;
3S-Amino-4-(1 H-imidazol-4-yl)-N-(5S-methyl-4-oxo-2-ureido-1,4,5,6-tetrahydro-pyrimidin-5-yl)-butyramide;
3R-Amino-4-(1H-imidazol-4-yl)-N-(5R-methyl-4-oxo-2-ureido-1,4,5,6-tetrahydro-pyrimidin-5-yl)-butyramide;
3R-Amino-4-(1H-imidazol-4-yl)-N-(5S-methyl-4-oxo-2-ureido-1,4,5,6-tetrahydro-pyrimidin-5-yl)-butyramide;
3R-Amino-6-guanidino-hexanoic acid methyl-(5S-methyl-4-oxo-2-ureido-1,4,5,6-tetrahydro-pyrimidin-5-yl)-amide;
3R-Amino-6-guanidino-hexanoic acid methyl-(5R-methyl-4-oxo-2-ureido-1,4,5,6-tetrahydro-pyrimidin-5-yl)-amide;
3S-Amino-4-(1H-indol-3-yl)-N-(5R-methyl-4-oxo-2-ureido-1,4,5,6-tetrahydro-pyrimidin-5-yl)-butyramide;
3S-Amino-4-(1H-indol-3-yl)-N-(5S-methyl-4-oxo-2-ureido-1,4,5,6-tetrahydro-pyrimidin-5-yl)-butyramide;
2S-Amino-5-guanidino-pentanoic acid (5R-methyl-4-oxo-2-ureido-1,4,5,6-tetrahydro-pyrimidin-5-yl)-amide;
2S-Amino-5-guanidino-pentanoic acid (5S-methyl-4-oxo-2-ureido-1,4,5,6-tetrahydro-pyrimidin-5-yl)-amide;
2R-Amino-5-guanidino-pentanoic acid (5R-methyl-4-oxo-2-ureido-1,4,5,6-tetrahydro-pyrimidin-5-yl)-amide;
2R-Amino-5-guanidino-pentanoic acid (5S-methyl-4-oxo-2-ureido-1,4,5,6-tetrahydro-pyrimidin-5-yl)-amide;
3R-Amino-6-guanidino-hexanoic acid (5S-hydroxymethyl-4-oxo-2-ureido-1,4,5,6-tetrahydro-pyrimidin-5-yl)-methyl-amide;
3R-Amino-6-guanidino-hexanoic acid (5R-hydroxymethyl-4-oxo-2-ureido-1,4,5,6-tetrahydro-pyrimidin-5-yl)-methyl-amide;
3R-Amino-6-guanidino-hexanoic acid (5R-fluoromethyl-4-oxo-2-ureido-1,4,5,6-tetrahydro-pyrimidin-5-yl)-methyl-amide; and,
3R-Amino-6-guanidino-hexanoic acid (5S-fluoromethyl-4-oxo-2-ureido-1,4,5,6-tetrahydro-pyrimidin-5-yl)-methyl-amide.

5. A pharmaceutical composition for the treatment of a disorder selected from a bacterial infection, a protozoal infection, and disorders related to bacterial infections or protozoal infections, in a mammal, fish, or bird which comprises a therapeutically effective amount of a compound according to claim 1 and a pharmaceutically acceptable carrier.

6. A pharmaceutical composition for the treatment of a disorder selected from a bacterial infection, a protozoal infection, and disorders related to bacterial infections or protozoal infections, in a mammal, fish, or bird which comprises a therapeutically effective amount of a compound according to claim 1 in combination with a beta-lactam, quinolone, tetracycline, streptogramin, aminoglycoside, glycopeptide, macrolide or oxazolidinone antibiotic; or in combination with a compound which inhibits bacterial or protozoal efflux or degradation of a compound according to claim 1.

7. A method of treating a disorder selected from a bacterial infection, a protozoal infection, and disorders related to bacterial infections or protozoal infections, in a mammal, fish, or bird which comprises administering to said mammal, fish or bird a therapeutically effective amount of a compound according to claim 1.

8. A method of treating a disorder selected from a bacterial infection, a protozoal infection, and disorders related to bacterial infections or protozoal infections, in a mammal, fish, or bird which comprises administering to said mammal, fish or bird a therapeutically effective amount of a compound according to claim 1 in combination or co-administered with a beta-lactam, quinolone, tetracycline, streptogramin, aminoglycoside, glycopeptide, macrolide or oxazolidinone antibiotic; or in combination with a compound which inhibits bacterial or protozoal efflux or degradation of a compound according to claim 1.
